(19) Europäisches Patentamt
European Patent Office
Office européen des brevets

(11) **EP 4 292 472 A1**

(12) **EUROPEAN PATENT APPLICATION**

(43) Date of publication:
**20.12.2023 Bulletin 2023/51**

(21) Application number: **22179367.2**

(22) Date of filing: **16.06.2022**

(51) International Patent Classification (IPC):
**A46B 15/00** (2006.01)    **B26B 21/40** (2006.01)
**G16H 40/63** (2018.01)

(52) Cooperative Patent Classification (CPC):
**A46B 15/0002; G16H 40/63;** A46B 2200/1046;
A46B 2200/1066; B26B 21/4056; G16H 20/30;
G16H 30/40; G16H 50/20; G16H 50/70

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **KULKARNI, Nikhil Vikram
  Eindhoven (NL)**
• **PATIL, Ravindra
  Eindhoven (NL)**

(74) Representative: **Philips Intellectual Property &
Standards
High Tech Campus 52
5656 AG Eindhoven (NL)**

(54) **ORAL HEALTH CARE**

(57)    Proposed concepts thus aim to provide schemes, solutions, concept, designs, methods and systems pertaining to assisting an oral health care routine of a user. It has been realized that captured video of the user and/or a personal care device during performance of an oral health care routine may be analysed to obtain motion data that may then be leveraged to determine at least one parameter value of the personal health care routine. That is, insights may be derived into the user's performance of an oral health care routine based on movements of their body and/or a personal care device, such as a toothbrush. Such video may be obtained using existing or conventional devices that include cameras already owned by a user.

FIG. 1

EP 4 292 472 A1

**Description**

FIELD OF THE INVENTION

This invention relates to the field of oral health care routines, and in particular to the field of assisting an oral health care routine of a user.

BACKGROUND OF THE INVENTION

Oral care devices, such as electric toothbrushes or mouthpieces, are used on a regular (e.g. daily) basis, and the oral health care routines involving these devices often involve several complex steps. Thus, the user performing the oral health care routine is often unable to judge their own performance of the routine accurately. A particularly problematic example is that of proper brushing of the teeth.

Guided brushing is a feature for toothbrush users to provide them with better oral care. Users are often prone to bias, brushing one side of their mouth more than the other, or focusing on the front teeth at the expense of the back. Better oral care can thus be achieved by providing to the users important brushing information, such as total time spent brushing each region of the mouth, or whether basic oral hygiene procedures have taken place, such as flossing. This information can be especially useful for parents keeping track of their children's dental cleaning routine.

Currently, guided brushing is offered using sensors embedded within the toothbrush such as gyroscopes. However, these sensors are expensive and hence only available in high-end, expensive toothbrushes. Most conventional devices for oral care do not include any sensors that would be suitable for monitoring an oral health care routine.

SUMMARY OF THE INVENTION

The invention is defined by the claims.

According to examples in accordance with an aspect of the invention, there is provided a method for assisting an oral health care routine of a user.

The method comprises: obtaining video data from captured video of the user performing the oral health care routine using a personal care device; processing the video data to obtain motion data describing motion of at least one of: the personal care device, and a portion of the user during performance of the oral health care routine; and analysing the motion data to determine at least one parameter value of the oral health care routine.

Proposed concepts thus aim to provide schemes, solutions, concept, designs, methods and systems pertaining to assisting an oral health care routine of a user.

In particular, embodiments aim to determine at least one parameter value of an oral health care routine based on the motion of a personal care device and/or a portion of the user during performance of the oral health care routine. Information on the motion of a personal care device and/or a portion of the user may be obtained from video of the user and/or the personal care device captured during performance of an oral health care routine. That is, in an example of a user brushing their teeth, video may be captured of the user brushing their teeth and then analysed. From analysis of the movements of the user and/or their toothbrush, insights into how well they brushed their teeth, i.e. spending the right amount of time on each tooth, may be derived. Guidance may then be offered to the user informing them on the quality of their performance.

In other words, it is proposed that captured video of the user and/or a personal care device during performance of an oral health care routine may be analysed to obtain motion data that may then be leveraged to determine at least one parameter value of the personal health care routine. That is, insights may be derived into the user's performance of an oral health care routine based on movements of their body and/or a personal care device, such as a toothbrush. Such video may be obtained using existing or conventional devices that include cameras already owned by a user.

By providing a computer vision based method of analysing oral health care routines, feedback analogous to guided brushing may be provided to users irrespective of the type of the toothbrush being used. However, embodiments are not limited to just toothbrushes, electric or manual. The personal care device may also comprise a mouthpiece, a flossing device, or any other personal oral care device. One or more proposed concept(s) may therefore be employed in a range of different personal care devices. Embodiments may therefore have wide application in the field of personal care devices, and be of particular relevance to dentistry propositions. For example, by enabling improved cleaning of a user's teeth, gum, tongue, etc. any by reducing unwanted tissue damage. Accordingly, embodiments may be used in relation to dental treatment so as to support a dental care professional when providing treatment for a subject.

By being integrated into the normal brushing regiment of a user, embodiments may support improved dental care. Improved oral health care routines may therefore be provided by proposed concepts.

For instance, by automatically analysing the motion of a personal care device or a portion of a user during performance of an oral health care routine, one or more insights or statistics may be determined that may be of use to the user. An example parameter value may, for instance, pertain to user bias. For instance, if the oral health care routine is the user brushing their teeth, the parameter value may be a ratio of time spent brushing the left side of the mouth to time spent brushing the right side of the mouth, or alternatively, percentage of the mouth cleaned. Such insights may enable the provision of feedback to the user, such that favourable habits are reinforced and unfavourable behaviour highlighted.

The use of video data alone to derive insights into a user's performance of an oral health care routine may permit assistance to the user in their routine without the need for dedicated sensors in the personal care device which are costly and increase the complexity of the required personal care device. Thus, assistance may be provided to users performing oral health care routines regardless of whether or not the personal care device involved has sensors suitable for monitoring an oral health care routine.

Ultimately, an improved performance of an oral health care routine by a user may be supported by the proposed concept(s).

In some embodiments, the at least one parameter value may comprise at least one of: a user bias; a measure of completion of the oral health care routine; a measure of completion of a subroutine of the oral health care routine; and a time duration. An example of user bias may be a ratio of time spent brushing the upper teeth to the time spent brushing the lower teeth. An example of a measure of completion of the oral health care routine may be a number of teeth remaining to be properly cleaned. An example of a measure of completion of a subroutine of the oral health care routine may be whether the user has flossed. An example of time duration may be the total time spent brushing each region of the mouth. These parameter values may enable the provision of feedback to the user, such that they become aware of deficiencies in their performance of the oral health care routine, enabling conscious improvement.

In some embodiments, processing the video data to obtain motion data describing motion of the personal care device may comprise: providing the video data as input to a first convolutional neural network, CNN, the first CNN being trained to predict, for the personal care device associated with the video data, motion data indicating a series of locations of the personal care device.

The use of a CNN instead of a plain object detection algorithm may allow pixel level segmentation of the personal care device compared to a simple bounding box. This may facilitate more accurate location and movement tracking of the personal care device.

In some embodiments, the series of locations of the personal care device may describe a region of the personal care device. For example, the location and motion of the top of a toothbrush, e.g. the brushing head, may be of specific use during analysis of the motion data to determine parameter values of the oral health care routine.

In some embodiments, the first CNN may be trained using a training algorithm configured to receive an array of training inputs and respective known outputs, wherein a training input comprises video data associated with a personal care device and respective known output comprises motion data indicating a series of locations of the personal care device. In this way, the first CNN may be trained to output motion data indicating a series of locations of the personal care device when provided with video data associated with a personal care device during an oral health care routine.

In some embodiments, the first CNN is a pretrained model further trained on videos of subjects using the personal care device that have been manually annotated. This allows the CNN to become especially proficient at identifying the personal care device.

In some embodiments, processing the video data to obtain motion data describing motion of a portion of the user comprises:

> providing the video data as input to a second neural network, the second neural network being trained to predict, for the portion of the user associated with the video data, motion data indicating a series of locations of a palm of a hand of the user;
> providing the video data as input to a third neural network, the third neural network being trained to predict, for the portion of the user associated with the video data, motion data indicating a series of locations of landmarks on a hand of the user.

This split model, separately identifying the location of a palm of a hand of the user and locations of landmarks on a hand of the user allows quick and accurate hand tracking without the use of any specialized hardware like a depth-perception camera. The third neural network can identify the locations of landmarks on a hand quickly by only searching within the bounding box identified by the second neural network.

In some embodiments, the second neural network is trained using a training algorithm configured to receive an array of training inputs and respective known outputs, wherein a training input comprises video data associated with a portion of the user and respective known output comprises motion data indicating a series of locations of a palm of a hand of the user. This allows the second neural network to become proficient at identifying palms of users.

And further, the third neural network is trained using a training algorithm configured to receive an array of training inputs and respective known outputs, wherein a training input comprises video data associated with a portion of the user and respective known output comprises motion data indicating a series of locations of landmarks on a hand of the user. This allows the third neural network to become proficient at identifying landmarks on a hand of a user.

In some embodiments, wherein the second neural network comprises single shot multibox detector architecture, and the third neural network comprises a feature pyramid network. Single shot multibox detector architecture allows multiple objects present in an image to be detected in single forward pass of the network. This allows multiple objects, such as two palms, to be detected quickly. Feature pyramid networks are especially proficient at identifying small objects, such as knuckles or finger joints, and so are well suited to detecting landmarks on a hand of a user.

In some embodiments, processing the video data to obtain motion data describing motion of a portion of the user comprises:

providing the video data as input to a fourth neural network, the fourth neural network being trained to predict, for the portion of the user associated with the video data, motion data indicating a series of locations of a face of the user;

providing the video data as input to a fifth neural network, the fifth neural network being trained to predict, for the portion of the user associated with the video data, motion data indicating a series of locations of landmarks on a face of the user.

This split model, separately identifying the location of a face of the user and locations of landmarks on the face of the user allows quick and accurate face tracking without the use of any specialized hardware like a depth-perception camera. By first identifying the face of the user, the landmarks on the face can then be identified quicker by the fifth neural network by only searching within the bounding box detected by the fourth neural network.

In some embodiments, the fourth neural network is trained using a training algorithm configured to receive an array of training inputs and respective known outputs, wherein a training input comprises video data associated with a portion of the user and respective known output comprises motion data indicating a series of locations of a face of the user. This allows the fourth neural network to become proficient at identifying faces of users.

And further, the fifth neural network is trained using a training algorithm configured to receive an array of training inputs and respective known outputs, wherein a training input comprises video data associated with a portion of the user and respective known output comprises motion data indicating a series of locations of landmarks on a face of the user. This allows the fifth neural network to become proficient at identifying landmarks on faces of users.

In some embodiments, the fourth neural network comprises single shot multibox detector architecture, and the fifth neural network comprises a feature pyramid network. Single shot multibox detector architecture allows multiple objects present in an image to be detected in single forward pass of the network. This allows single or multiple objects, such as one or more faces, to be detected quickly. Feature pyramid networks are especially proficient at identifying small objects, such as nostrils and eyes, and so are well suited to detecting landmarks on a face of a user.

In some embodiments, analysing the motion data to determine at least one parameter value of the oral health care routine comprises:
providing the motion data as input to a machine learning algorithm, the machine learning algorithm being trained to predict, for the oral health care routine associated with the motion data, at least one parameter value of the oral health care routine. From the personal care device locations, hand and facial landmark locations, and pattern matching, the machine learning algorithm can deduce useful information. For example, in the case of a user brushing their teeth, the machine learning algorithm can predict the angle of the personal care device with respect to the user's mouth.

In some embodiments, the machine learning algorithm comprises a supervised classifier model. This is especially useful for classifying detected behaviour, such as brushing specific regions of a user's mouth.

In some embodiments, the machine learning algorithm is trained using a training algorithm configured to receive an array of training inputs and respective known outputs, wherein a training input comprises motion data associated with an oral health care routine and respective known output comprises at least one parameter value of the oral health care routine. This allows the machine learning algorithm to become especially proficient at determining parameter values of oral health care routines.

In some embodiments, analysing the motion data to determine at least one parameter value of the oral health care routine comprises:
providing the motion data as input to a rule-based algorithm designed to predict, for the oral health care routine associated with the motion data, at least one parameter value of the oral health care routine. This allows parameter values to be deduced from the motion data without the use of a machine learning algorithm which may ease computational demands.

In some embodiments, analysing the motion data to determine at least one parameter value of the oral health care routine comprises:
predicting a location of contact between the personal care device and a surface of the user. For example, in the case of a user brushing their teeth, from the location of contact between the personal care device and a surface of the user, the region of brushing taking place can be deduced. For instance, it can be deduced the user is currently brushing their left molar.

In some embodiments, analysing the motion data to determine at least one parameter value of the oral health care routine comprises:

predicting a distance between each palm of the user and the face of the user,
and optionally wherein the predicted distances between each palm of the user and the face of the user are compared to predetermined thresholds.

This allows the detection of a user flossing, as they will need both hands close to their mouth while flossing.

In some embodiments, there is provided a computer program comprising code means for implementing any of the methods described above when said program is run on a processing system. According to another aspect of the invention, there is provided a system for assisting an oral health care routine of a user, the system comprises:

an input interface configured to obtain video data from captured video of the user performing the personal health care routine using a personal care device;

a processor arrangement configured to:

process the video data to obtain motion data describing

motion of at least one of the personal care device and a portion(s) of the user during performance of the oral health care routine;

analyse the motion data to determine at least one parameter value of the oral health care routine.

Thus, there may be proposed concepts for assisting an oral health care routine of a user, and this may done based on visually-observed motion of the user during performance of the oral health care routine. Determining parameter values of the oral health care routine may help to inform the user on the quality of their performance of the oral health care routine and allow for them to adjust their performance accordingly.

These and other aspects of the invention will be apparent from and elucidated with reference to the embodiment(s) described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a simplified flow diagram of a method for assisting an oral health care routine of a user according to a proposed embodiment;

Fig. 2 is a more in-depth flow diagram of a method for assisting an oral health care routine of a user according to a proposed embodiment;

Fig. 3a is a simplified diagram of tracking a personal care device according to a proposed embodiment;

Fig. 3b is a simplified diagram of tracking a personal care device at an alternate angle according to a proposed embodiment;

Fig. 4 is a simplified block diagram of a system for assisting an oral health care routine of a user according a proposed embodiment; and

Fig. 5 illustrates an example of a computer within which one or more parts of an embodiment may be employed

DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to assisting an oral health care routine of a user. According to proposed concepts, a number of possible solutions may be implemented separately or jointly. That is, although these possible solutions may be described below separately, two or more of these possible solutions may be implemented in one combination or another.

Embodiments of the invention aim to assist an oral health care routine of a user by analysing motion (i.e. movement) of a user and a personal care device during performance of the oral health care routine. This can be achieved by obtaining video data from captured video of the user performing the oral health care routine using a personal care device. The video data can then be processed to obtain motion data describing motion of at least one of: the personal care device, and a portion of the user during performance of the oral health care routine. The motion data can then be analysed to determine at least one parameter value of the oral health care routine.

Proposed concepts thus aim to determine at least one parameter value of an oral health care routine based on the motion of a personal care device and/or a portion of the user during performance of the oral health care routine. Information on the motion of a personal care device and/or a portion of the user may be obtained from video of the user and/or the personal care device captured during performance of an oral health care routine. That is, in an example of a user brushing their teeth, video may be captured of the user brushing their teeth and then analysed. From analysis of the movements of the user and/or their toothbrush, insights into how well they brushed their teeth, i.e. spending the right amount of time on each tooth, may be derived. Guidance may then be offered to the user informing them on the quality of their performance.

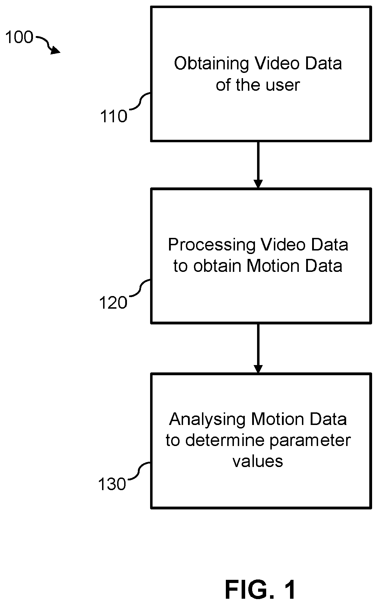

Referring now to Fig. 1, there is depicted a flow diagram of a method 100 for assisting an oral health care routine of a user according to a proposed embodiment.

The method begins with the step 110 of obtaining video data from captured video of the user performing the oral health care routine using a personal care device. Such video can be obtained using existing or conventional devices that include cameras already owned by a user. For instance, a conventional device may be one of: a smartphone, a tablet, a laptop, or any other suitable device. By providing a computer vision based method of analysing oral health care routines, feedback analogous to guided brushing may be provided to users irrespective of the type of the toothbrush or personal care device being used. The use of video data alone to derive insights into a user's performance of an oral health care routine may permit assistance to the user in their routine without the need for dedicated sensors in the personal care device which are costly and increase the complexity of the required personal care device. Thus, assistance may be provided to users performing oral health care routines regardless of whether or not the personal care device involved has sensors suitable for monitoring an oral health care routine.

In step 120, the video data is processed to obtain motion data describing motion of at least one of: the personal care device, and a portion of the user during performance of the oral health care routine. The details of the processing of the video data will be more fully explained later.

In step 130, the motion data is analysed to determine at least one parameter value of the oral health care routine. An example parameter value may, for instance, pertain to user bias. For instance, if the oral health care routine is the user brushing their teeth, the parameter value may be a ratio of time spent brushing the left side of the mouth to time spent brushing the right side of the mouth, or alternatively, percentage of the mouth cleaned. Such insights may enable the provision of feedback to the user, such that favourable habits are reinforced and unfavourable behaviour highlighted.

In some embodiments, the at least one parameter value may comprise at least one of: a user bias; a measure of completion of the oral health care routine; a measure of completion of a subroutine of the oral health care routine; and a time duration. An example of user bias may be a ratio of time spent brushing the upper teeth to the time spent brushing the lower teeth. An example of a measure of completion of the oral health care routine may be a number of teeth remaining to be properly cleaned. An example of a measure of completion of a subroutine of the oral health care routine may be whether the user has flossed. An example of time duration may be the total time spent brushing each region of the mouth. These parameter values may enable the provision of feedback to the user, such that they become aware of deficiencies in their performance of the oral health care routine, enabling conscious improvement.

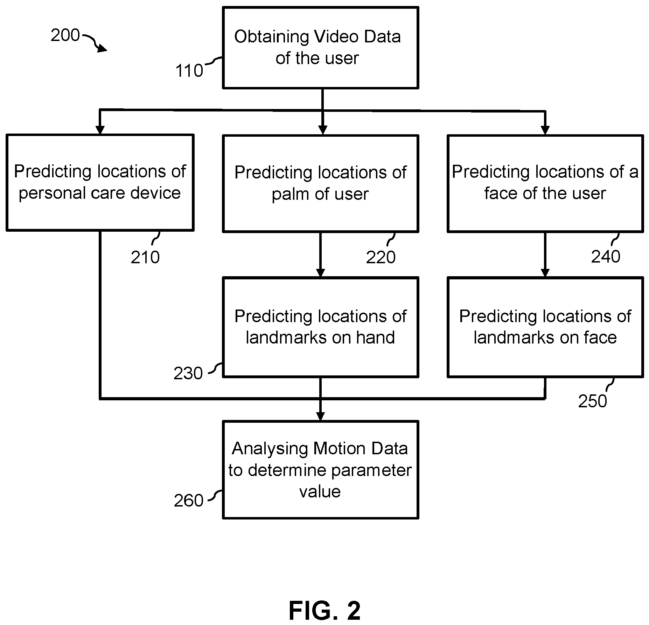

Referring now to Fig. 2, there is depicted a more in-depth flow diagram of a method 200 for assisting an oral health care routine of a user according to an exemplary embodiment. The method begins with the step 110 of obtaining video data from captured video of the user performing the oral health care routine using a personal care device, as described above.

In step 210, the video data is processed to predict motion data indicating a series of locations of the personal care device. The processing of the video data is facilitated by providing the video data as input to a first convolutional neural network, CNN, the first CNN being trained to predict, for the personal care device motion data indicating a series of locations of the personal care device. In this way, captured movement of the personal care device during performance of the oral health care routine is converted/translated into motion data.

The use of a CNN instead of a plain object detection algorithm allows pixel level segmentation of the personal care device compared to a simple bounding box. This may facilitate more accurate location and movement tracking of the personal care device.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

There are several types of neural network, such as convolutional neural networks (CNNs) and recurrent neural networks (RNNs). This exemplary embodiment of the present invention employs CNN-based learning algorithms, because CNNs have proved to be particularly successful at analyzing videos, and are able to classify frames within videos with a much lower error rate than other types of neural network.

CNNs typically contain several layers, including a convolutional layer, a pooling layer, and a fully connected layer. The convolutional layer consists of a set of learnable filters and extracts features from the input. The pooling layer is a form of non-linear down-sampling, reducing the data size by combining the outputs of a plurality of neurons in one layer into a single neuron in the next layer. The fully connected layer connects each neuron in one layer to all the neurons in the next layer.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, weightings of the mathematical operation of each neuron may be modified until the error converges. Known methods of modifying a neural network include gradient descent, backpropagation algorithms and so on.

The training input data entries for the first CNN used in method 200 correspond to example video data associated with a personal care device. The training output data entries correspond to motion data indicating a series of locations of the personal care device. Further, several pre-processing methods may be employed to improve the training samples. In other words, the first CNN can be trained using a training algorithm configured to receive an array of training inputs and respective known outputs, wherein a training input comprises video data associated with a personal care device and respective known output comprises motion data indicating a series of locations of the personal care device. In this way, the first CNN is trained to output motion data indicating a series of locations of the personal care device when provided with video data associated with a personal care device during an oral health care routine.

In some embodiments, the first CNN is a pretrained model further trained on videos of subjects using the personal care device that have been manually annotated. This allows the CNN to become especially proficient at identifying the personal care device. In some embodiments, the pretrained model is trained on a COCO dataset based on RESNET34, RESNET 50, and/or RESNET101 backbone architecture.

In some embodiments, the first CNN is more specifically a Mask-RCNN deep neural based model trained on custom toothbrush annotations.

In some embodiments, the series of locations of the personal care device describes a region of the personal care device. For example, the location and motion of the top edge of a toothbrush, e.g. the brushing head, may be of specific use during analysis of the motion data to determine parameter values of the oral health care routine.

In step 220, the video data is processed to predict motion data indicating a series of locations of a portion of the user during performance of the oral health care routine. In step 220, the portion of the user is a palm of a hand of the user. The processing is facilitated by providing the video data as input to a second neural network, the second neural network being trained to predict, for the portion of the user associated with the video data, motion data indicating a series of locations of a palm of a hand of the user. In an embodiment, the second neural network comprises single shot multibox detector architecture. Single shot multibox detector architecture allows multiple objects present in an image to be detected in single forward pass of the network. This allows multiple objects, such as two palms, to be detected quickly.

The second neural network is trained using a training algorithm configured to receive an array of training inputs and respective known outputs, wherein a training input comprises video data associated with a portion of the user and respective known output comprises motion data indicating a series of locations of a palm of a hand of the user. This allows the second neural network to become proficient at identifying palms of users and drawing bounding boxes around them.

In step 230, the video data is processed to predict motion data indicating a series of locations of a portion of the user during performance of the oral health care routine. In step 230, the portion of the user is locations of landmarks on a hand of the user. The processing is facilitated by providing the video data as input to a third neural network, the third neural network being trained to predict, for the portion of the user associated with the video data, motion data indicating a series of locations of landmarks on a hand of the user. In an embodiment, the third neural network comprises a feature pyramid network. Feature pyramid networks are especially proficient at identifying small objects, such as knuckles or finger joints, and so are well suited to detecting landmarks on a hand of a user. In an embodiment, at least 21 landmarks on the hand are detected.

The third neural network is trained using a training algorithm configured to receive an array of training inputs and respective known outputs, wherein a training input comprises video data associated with a portion of the user and respective known output comprises motion data indicating a series of locations of landmarks on a hand of the user. This allows the third neural network to become proficient at identifying landmarks on a hand of a user. In an embodiment, the coordinates of identified landmarks on a hand of a user are saved to a memory for further analysis.

This split model, separately identifying the location of a palm of a hand of the user and locations of landmarks on a hand of the user allows quick and accurate hand tracking, for instance tracking of a hand skeleton, without the use of any specialized hardware like a depth-perception camera. The third neural network can identify the locations of landmarks on a hand quickly by only searching within the bounding box identified by the second neural network. The landmarks can be identified using encoder-decoder architecture.

In step 240, the video data is processed to predict motion data indicating a series of locations of a portion of the user during performance of the oral health care routine. In step 240, the portion of the user is a face of the user. The processing is facilitated by providing the video data as input to a fourth neural network, the fourth neural network being trained to predict, for the portion of the user associated with the video data, motion data indicating a series of locations of a face of the user. In an embodiment, the fourth neural network comprises single shot multibox detector architecture. Single shot multibox detector architecture allows multiple objects present in an image to be detected in single forward pass of the network. This allows single or multiple objects, such as one or more faces, to be detected quickly.

The fourth neural network is trained using a training algorithm configured to receive an array of training inputs and respective known outputs, wherein a training input comprises video data associated with a portion of the user and respective known output comprises motion data indicating a series of locations of a face of the user. This allows the fourth neural network to become proficient at identifying faces of users.

In step 250, the video data is processed to predict motion data indicating a series of locations of a portion of the user during performance of the oral health care routine. In step 250, the portion of the user is locations of landmarks on a face of the user. The processing is facilitated by providing the video data as input to a fifth neural network, the fifth neural network being trained to predict, for the portion of the user associated with the video data, motion data indicating a series of locations of landmarks on a face of the user. In an embodiment, the fifth neural network comprises a feature pyramid network. Feature pyramid networks are especially proficient at identifying small objects, such as nostrils and eyes, and so are well suited to detecting landmarks on a face of a user. In an embodiment, at least 230 landmarks on the face are detected.

The fifth neural network is trained using a training algorithm configured to receive an array of training inputs and respective known outputs, wherein a training input comprises video data associated with a portion of the user and respective known output comprises motion data indicating a series of locations of landmarks on a face of the user. This allows the fifth neural network to become proficient at identifying landmarks on faces of users. In an embodiment, the coordinates of identified landmarks on a face of a user are saved to a memory for further analysis.

This split model, separately identifying the location of a face of the user and locations of landmarks on the face of the user allows quick and accurate face tracking without the use of any specialized hardware like a depth-perception camera. By first identifying the face of the user, the landmarks on the face can then be identified quicker by the fifth neural network by only searching within the bounding box detected by the fourth neural network. The landmarks can be identified using encoder-decoder architecture.

In step 260, the motion data determined by steps 210, 220, 230, 240, and 250 is analysed to determine at least one parameter value of the oral health care routine. This can be done one of two ways:

i.) The motion data may be provided as input to a machine learning algorithm, the machine learning algorithm being trained to predict, for the oral health care routine associated with the motion data, at least one parameter value of the oral health care routine. From the personal care device locations, hand and facial landmark locations, and pattern matching, the machine learning algorithm can deduce useful information. For example, in the case of a user brushing their teeth, the machine learning algorithm can predict the angle of the personal care device with respect to the user's mouth.

In an embodiment, the machine learning algorithm comprises a supervised classifier model. This is especially useful for classifying detected behaviour, such as brushing specific regions of a user's mouth. Region of brushing can be deduced based on personal care device, hand and facial landmarks coordinates and pattern matching.

The machine learning algorithm is trained using a training algorithm configured to receive an array of training inputs and respective known outputs, wherein a training input comprises motion data associated with an oral health care routine and respective known output comprises at least one parameter value of the oral health care routine. This allows the machine learning algorithm to become especially proficient at determining parameter values of oral health care routines.

ii.) The motion data may be provided as input to a rule-based algorithm designed to predict, for the oral health care routine associated with the motion data, at least one parameter value of the oral health care routine. This allows parameter values to be deduced from the motion data without the use of a machine learning algorithm which may ease computational demands. For instance, if a toothbrush head is closer to the right edge of a mouth than the left, and the angle between the toothbrush and a line parallel to a plane of the face is 95 degrees then it can be determined that brushing is taking place on the right side of the mouth.

In one embodiment, analysing the motion data to determine at least one parameter value of the oral health care routine by either method comprises predicting a location of contact between the personal care device and a surface of the user. For example, in the case of a user brushing their teeth, from the location of contact between the personal care device and a surface of the user, the region of brushing taking place can be deduced.

For instance, it can be deduced that the user is currently brushing their left molar.

In one embodiment, analysing the motion data to determine at least one parameter value of the oral health care routine comprises predicting a distance between each palm of the user and the face of the user. The predicted distance between each palm of the user and the face of the user can then be compared to predetermined thresholds. This allows the detection of a user flossing, as they will need both hands close to their mouth while flossing, but is not limited to this purpose.

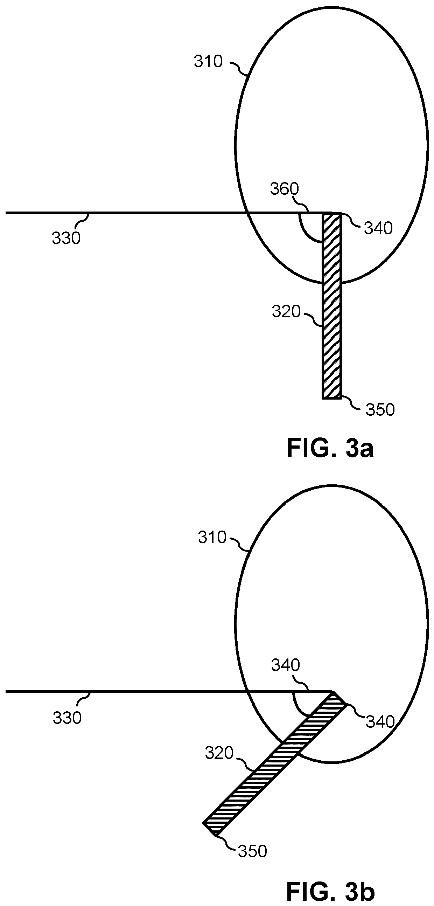

Referring now to Fig. 3a and Fig. 3b, there are depicted simplified diagrams of tracking a personal care device according to a proposed embodiment. These diagrams depict a user performing an oral health care routine, for example, brushing their teeth. In both diagrams, a personal care device 320 is being held up to the face 310 of a user. The top edge 340 of the personal care device and the bottom edge 350 are used to find an angle 360 of the personal care device against a line 330 parallel to the plane of the face and going through the top edge.

From processing of the video data to obtain motion data indicating a series of locations of the personal care device 320, the first CNN outputs a mask of the personal care device. In this example, the personal care device is a toothbrush. From the outputted mask, a top edge 340 (i.e. a Y-coordinate minimum where the top of the video frame is Y=0) and a bottom edge 350 (i.e. a Y-coordinate maximum) of the toothbrush can be found.

In an embodiment, the first CNN is a Mask-RCNN, but in this embodiment, the mask of the personal care device 320 generated by the Mask-RCNN can be made up of many sub masks. To overcome this issue and to determine the top edge 340 and bottom edge 350 of the personal care device, the following algorithm is implemented:
First, the top edge 340 is extracted by finding the minimum Y-coordinate (where the top of the video frame is Y=0) of the mask and by finding its corresponding X-coordinate. Second, the bottom edge 350 is extracted by finding the maximum Y-coordinate of the mask and by finding its corresponding X-coordinate. Thirdly, if more than one mask of the personal care device 320 exists, then the top edge and the bottom edge of each sub-mask is found iteratively and the two prior steps are repeated to arrive at the top and bottom edges of the personal care device.

After the top edge 340 and the bottom edge 350 of the personal care device 320 have been found, the angle 360 of the personal care device with respect to the plane 330 of the face 310 can be found. The angle between the toothbrush and the face is used to determine the location of brushing. For example, if the user is brushing the sides of their mouth then the toothbrush is almost perpendicular to the plane of the face, while if the user is brushing the front of the mouth then the toothbrush is nearly parallel to the face. To determine the angle, the top edge and the bottom edge of the toothbrush are connected with a straight line. This line then intersects the reference line 330 parallel to the plane of the face 310 at the top edge, which enables the angle between the two lines to be determined using dot product. This angle is equal to the angle between the toothbrush and the face.

Treating the two lines as two vectors, the angle between the two vectors can be computed using the following equation (i):

$$\theta = cos^{-1} \frac{\vec{a}.\vec{b}}{|\vec{a}||\vec{b}|} \qquad (i)$$

Wherein, $\vec{a}.\vec{b}$ is the dot product between the two vectors and $|\vec{a}||\vec{b}|$ are the vector's magnitude.

In an embodiment, the output coordinates of each first to fifth neural networks are saved to a memory. The saved coordinates are then analysed and the location of brushing is predicted using either the rule based or machine-learning based approach.

A memory can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory may incorporate electronic, magnetic, optical, and/or other types of storage media. Note that the memory can have a distributed architecture, where various components are situated remote from one another, but can be accessed by a processor.

In some embodiments, there is provided a computer program comprising code means for implementing any of the methods described above when said program is run on a processing system.

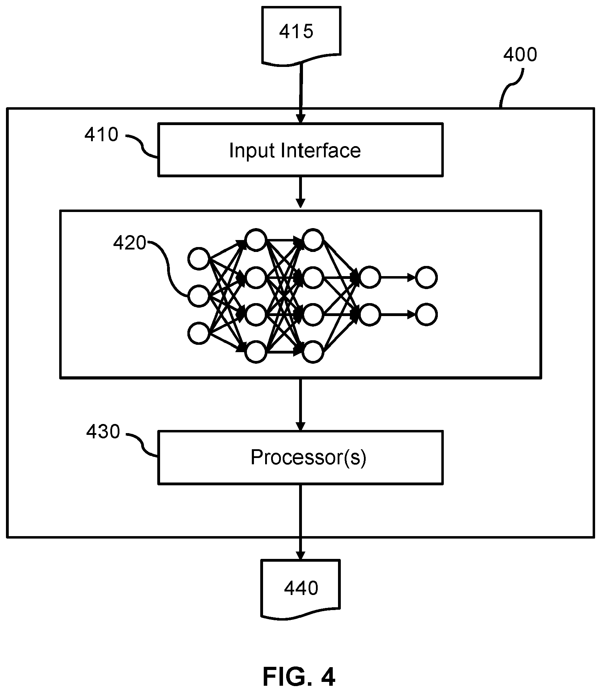

Referring now to Fig. 4, there is depicted a simplified block diagram of a system 400 for assisting an oral health care routine of a user according an embodiment. The system configured for assisting an oral health care routine of a user comprises an input interface 410, and one or more processors 430.

The system 400 is configured to analyse a user's motion, and the motion of a personal care device, during performance of an oral health care routine. Video 415 captured of the user performing the oral health care routine is obtained. The system outputs an output 450 comprising at least one parameter value of the oral health care routine. The output 450 is generated based on the analysis of the motion data, and is for providing feedback to the user on their performance of the oral health care routine.

In more detail, the input interface 410 receives video data 415 from captured video of the user during performance of an oral health care routine. The input interface 410 provides the video data 415 to one or more neural networks 420 for predicting locations of a portion(s) of the user within the video data, and for predicting locations of the personal health care device within the video data. This facilitates motion pattern analysis of an oral health care routine.

Based on the identified locations, the processor(s) 430 then processes the video data with a motion detection algorithm to obtain motion data describing motion of at least one of the personal care device and a portion(s) of the user during performance of the oral health care routine. For this, a multi-person detection network may be used to avoid external disturbances by focusing on the user performing the oral health care routine only.

The resulting motion data are then processed by the processor(s) 430 to produce an output 450 comprising at least one parameter value of the oral health care routine.

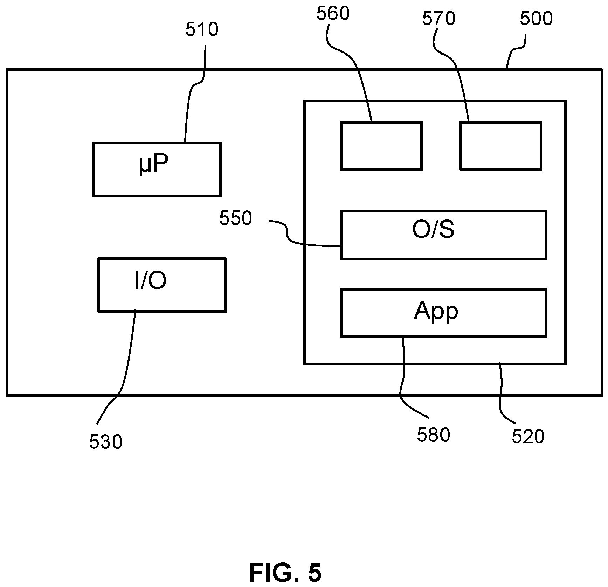

Figure 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 800 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-2, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Figure 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

**Claims**

1. A method for assisting an oral health care routine of a user, the method comprising:

   obtaining (110) video data from captured video of the user performing the oral health care routine using a personal care device;
   processing (120) the video data to obtain motion data describing motion of at least one of: the personal care device; and a portion of the user during performance of the oral health care routine; and
   analysing (130) the motion data to determine at least one parameter value of the oral health care routine.

2. The method of claim 1, wherein the at least one parameter value comprises at least one of: a user bias; a measure of completion of the oral health care routine; a measure of completion of a subroutine of the oral health care routine; and a time duration.

3. The method of any prior claim, wherein processing the video data to obtain motion data describing motion of the personal care device comprises:

   providing the video data as input to a first convolutional neural network, CNN, the first CNN being trained to predict, for the personal care device associated with the video data, motion data indicating a series of locations of the personal care device,
   and optionally wherein the series of locations of the personal care device describe a region of the personal care device.

4. The method of claim 3, wherein the first CNN is trained using a training algorithm configured to receive an array of training inputs and respective known outputs, wherein a training input comprises video data associated with a personal care device and respective known output comprises motion data indicating a series of locations of the personal care device.

5. The method of claim 3, wherein the first CNN is a pretrained model further trained on videos of subjects using the personal care device that have been manually annotated.

6. The method of any prior claim, wherein processing the video data to obtain motion data describing motion of a portion of the user comprises:

   providing the video data as input to a second neural network, the second neural network being trained to predict, for the portion of the user associated with the video data, motion data indicating a series of locations of a palm of a hand of the user;
   providing the video data as input to a third neural network, the third neural network being trained to predict, for the portion of the user associated with the video data, motion data indicating a series of locations of landmarks on a hand of the user.

7. The method of claim 6, wherein the second neural network is trained using a training algorithm configured to receive an array of training inputs and respective known outputs, wherein a training input comprises video data associated with a portion of the user and respective known output comprises motion data indicating a series of locations of a palm of a hand of the user;
   and wherein the third neural network is trained using a training algorithm configured to receive an array of training inputs and respective known outputs, wherein a training input comprises video data associated with a portion of the user and respective known output comprises motion data indicating a series of locations of landmarks on a hand of the user.

8. The method of claim 6 or 7, wherein the second neural network comprises single shot multibox detector architecture, and the third neural network comprises a feature pyramid network.

9. The method of any prior claim, wherein processing the video data to obtain motion data describing motion of a portion of the user comprises:

   providing the video data as input to a fourth neural network, the fourth neural network being trained to predict, for the portion of the user associated with the video data, motion data indicating a series of locations of a face of the user;
   providing the video data as input to a fifth neural network, the fifth neural network being trained to predict, for the portion of the user associated with the video data, motion data indicating a series of locations of landmarks on a face of the user.

10. The method of claim 9 wherein the fourth neural network is trained using a training algorithm configured to receive an array of training inputs and respective known outputs, wherein a training input comprises video data associated with a portion of the user and respective known output comprises motion data indicating a series of locations of a face of the user; and wherein the fifth neural network is trained using a training algorithm configured to receive an array of training inputs and respective known outputs, wherein a training input comprises video data associated with a portion of the user and respective known output comprises motion data indicating a series of locations of landmarks on a face of the user.

11. The method of claim 9 or 10, wherein the fourth neural network comprises single shot multibox detector architecture, and the fifth neural network comprises a feature pyramid network.

12. The method of any prior claim, wherein analysing the motion data to determine at least one parameter value of the oral health care routine comprises:

   providing the motion data as input to a machine learning algorithm, the machine learning algorithm being trained to predict, for the oral health care routine associated with the motion data, at least one parameter value of the oral health care routine,
   and optionally wherein the machine learning algorithm comprises a supervised classifier model.

13. The method of claim 12, wherein the machine learning algorithm is trained using a training algorithm configured to receive an array of training inputs and respective known outputs, wherein a training input comprises motion data associated with an oral health care routine and respective known output comprises at least one parameter value of the oral health care routine.

14. The method of any of claims 1-11, wherein analysing the motion data to determine at least one parameter value of the oral health care routine comprises: providing the motion data as input to a rule-based algorithm designed to predict, for the oral health care routine associated with the motion data, at least one parameter value of the oral health care routine.

15. The method of any previous claim, wherein analysing the motion data to determine at least one parameter value of the oral health care routine comprises: predicting a location of contact between the personal care device and a surface of the user.

16. The method of any previous claim, wherein analysing the motion data to determine at least one parameter value of the oral health care routine comprises:

   predicting a distance between each palm of the user and the face of the user,
   and optionally wherein the predicted distances between each palm of the user and the face of the user are compared to predetermined thresholds.

17. A computer program comprising code means for implementing the method of any preceding claim when said program is run on a processing system.

18. A system for assisting an oral health care routine of a user, the system comprising:

   an input interface (410) configured to obtain video data from captured video of the user performing the personal health care routine using a personal care device;
   a processor arrangement (430) configured to:

      process the video data to obtain motion data describing
      motion of at least one of the personal care device and a portion(s) of the user during performance of the oral health care routine;
      analyse the motion data to determine at least one parameter value of the oral health care routine.

FIG. 1

Obtaining Video Data
of the user

110

200

Predicting locations of
personal care device

210

Predicting locations of
palm of user

220

Predicting locations of a
face of the user

240

Predicting locations of
landmarks on hand

230

Predicting locations of
landmarks on face

250

Analysing Motion Data
to determine parameter
value

260

## FIG. 2

FIG. 3a

FIG. 3b

FIG. 4

**FIG. 5**

Europäisches
Patentamt

European
Patent Office

Office européen
des brevets

# EUROPEAN SEARCH REPORT

**Application Number**

EP 22 17 9367

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/201272 A1 (JOYCE ET AL.) 25 June 2020 (2020-06-25) * paragraphs [0131] – [0133], [0233] – [0225], [0259], [0265], [0268]; claims 1,14 * | 1-18 | INV. A46B15/00 B26B21/40 G16H40/63 |
| X | "Method and System for Measuring Effectiveness of Tooth Brushing ED – Darl Kuhn", IP.COM, IP.COM INC., WEST HENRIETTA, NY, US, 6 December 2018 (2018-12-06), XP013181263, ISSN: 1533-0001 * the whole document * | 1,17,18 | |
| X | WO 2021/197801 A1 (UNILEVER IP HOLDINGS ET AL.) 7 October 2021 (2021-10-07) * claims 1,2,11,12,19,20 * | 1,17,18 | |
| X | US 2018/132602 A1 (GATZEMEYER) 17 May 2018 (2018-05-17) * figures 1,1B * | 1,17,18 | TECHNICAL FIELDS SEARCHED (IPC) |
| X | US 2020/179089 A1 (SERVAL ET AL) 11 June 2020 (2020-06-11) * figures 1,2,4,7,9B * | 1,17,18 | A46B G16H B26B |

The present search report has been drawn up for all claims

| Place of search | Date of completion of the search | Examiner |
|---|---|---|
| The Hague | 25 October 2022 | Raybould, Bruce |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 17 9367

25-10-2022

| Patent document cited in search report | Publication date | Patent family member(s) | | Publication date |
|---|---|---|---|---|
| US 2020201272 A1 | 25-06-2020 | CN | 113168542 A | 23-07-2021 |
| | | CN | 113168909 A | 23-07-2021 |
| | | CN | 113168910 A | 23-07-2021 |
| | | CN | 113168911 A | 23-07-2021 |
| | | EP | 3899972 A2 | 27-10-2021 |
| | | EP | 3899973 A1 | 27-10-2021 |
| | | EP | 3899974 A1 | 27-10-2021 |
| | | EP | 3899984 A1 | 27-10-2021 |
| | | US | 2020201266 A1 | 25-06-2020 |
| | | US | 2020201272 A1 | 25-06-2020 |
| | | US | 2020202520 A1 | 25-06-2020 |
| | | US | 2020202521 A1 | 25-06-2020 |
| | | WO | 2020131471 A2 | 25-06-2020 |
| | | WO | 2020131472 A1 | 25-06-2020 |
| | | WO | 2020131473 A1 | 25-06-2020 |
| | | WO | 2020131474 A1 | 25-06-2020 |
| WO 2021197801 A1 | 07-10-2021 | BR | 112022016783 A2 | 11-10-2022 |
| | | WO | 2021197801 A1 | 07-10-2021 |
| US 2018132602 A1 | 17-05-2018 | AU | 2017356206 A1 | 30-05-2019 |
| | | CA | 3037374 A1 | 17-05-2018 |
| | | CN | 108066029 A | 25-05-2018 |
| | | EP | 3525619 A1 | 21-08-2019 |
| | | RU | 2712338 C1 | 28-01-2020 |
| | | US | 2018132602 A1 | 17-05-2018 |
| | | US | 2022079329 A1 | 17-03-2022 |
| | | WO | 2018089886 A1 | 17-05-2018 |
| US 2020179089 A1 | 11-06-2020 | AU | 2017316731 A1 | 21-03-2019 |
| | | BR | 112019003640 A2 | 21-05-2019 |
| | | CN | 110213980 A | 06-09-2019 |
| | | EP | 3493701 A1 | 12-06-2019 |
| | | RU | 2019106205 A | 22-09-2020 |
| | | US | 2020179089 A1 | 11-06-2020 |
| | | US | 2022249214 A1 | 11-08-2022 |
| | | WO | 2018037318 A1 | 01-03-2018 |

EPO FORM P0459